# DEMANDE DE BREVET EUROPEEN

(11) **EP 2 468 951 A1**
(43) Date de publication de la demande: **27.06.2012**
(21) Numéro de dépôt: 10196305.6
(22) Date de dépôt: 21.12.2010
(51) Int. Cl.: D21C 5/00, C12N 9/00

(54) **Procédé de traitement de matières cellulosiques**

(71) Demandeur: REALCO S.A., 1348 Louvain-la-Neuve (BE); Celodev SARL, 51260 Anglure (FR)
(72) Inventeur: Blackman, Gordon, 1380 Lasnes (BE); Boels, Gauthier, 1040 Bruxelles (BE); Gathy, Camille, 1341 Céroux-Mousty (BE); Bajul, Gilles, 51120 Villeneuve Saint Vistre (FR)
(74) Mandataire: Coulon, Ludivine

(57) **Abrégé**

Procédé de traitement de matière cellulosiques comprenant une addition d'une ou de plusieurs enzymes ayant une activité dans le traitement susdit desdites matières cellulosiques, une inhibition de ladite activité enzymatique qui est une inhibition à la demande et réversible.

## Description

La présente invention se rapporte à un procédé de traitement de matières cellulosiques pour fabriquer du papier, du carton ou de la pâte à papier comprenant les étapes suivantes :
- addition d'une ou de plusieurs enzymes ayant une activité dans le traitement susdit desdites matières cellulosiques, et
- inhibition de ladite activité enzymatique.

Dans les procédés de fabrication de papier, il existe trois grands types d'acteurs sur le marché.

Le premier type d'acteur concerne les fabricants de pâte à papier qui, à partir d'une matière cellulosique première (fibres cellulosiques végétales,à savoir le bois, les tissus, les plantes annuelles) effectuent la séparation des fibres cellulosiques. Les fibres cellulosiques sont amalgamées ensemble et recouvertes de lignine. Pour obtenir ces fibres cellulosiques séparées, des copeaux de matière cellulosique première, par exemple de bois ou de fibres par exemple de plantes annuelles (tiges de lin, chanvre, cotton, etc.) peuvent être broyées dans des raffineurs, de préférence en présence de vapeur. A la sortie des raffineurs, les copeaux issus du broyage sont mélangés avec de l'eau. On appelle cette étape le pulpage (thermo-mécanique). Dans d'autres cas, les copeaux sont autoclavés en présence de produits chimiques, ce qui permet de dissoudre les lignines et de libérer les fibres de cellulose. On appelle également cette étape le pulpage (chimique) qui permet de former une pâte à papier chimique.

Après pulpage de la matière première cellulosique, la pulpe subit une séparation solide-liquide et la fraction solide est appelée la pâte à papier. Cette pate à papier est alors conditionnée en ballot ou balles et constitue le produit commercialisé par ce premier type d'acteurs sur le marché.

Le deuxième type d'acteur concerne les fabricants de papier ou de carton qui s'approvisionnent en ballot ou balles de pâte à papier et effectuent la fabrication du papier ou du carton. A partir de ces balles, la pâte à papier séchée acheminée est alors pulpée en présence d'eau avant traitement mécanique et/ou chimique. Parmi ce deuxième type d'acteurs sur le marché, on trouve aussi les utilisateurs de papiers recyclés qui effectuent leur propre pâte à papier à partir de papier recyclé. Dans ce cas, les papiers et cartons recyclés sont broyés et ensuite mélangés à de l'eau. Les contaminants éventuels sont alors éliminés comme les plastiques, le verre ou encore des résidus métalliques. Comme on le comprend, le terme pulpage, tel qu'utilisé ici, dans le contexte de la présente demande de brevet, couvre toute étape d'une matière cellulosique mise en contact avec de l'eau que ce soit la matière première cellulosique (bois, fibre,...) ou la pâte à papier séchée des ballots.

Généralement, le traitement physique et/ou chimique comprend plusieurs traitements en séquence dont certains peuvent être chimiques et d'autres mécaniques. Dans la majorité des cas, une étape mécanique de raffinage est appliquée qui comprend une injection de la pâte sous pression entre des roues cannelées d'un raffineur afin de déstructurer et déchirer les fibres avant stockage dans une cuve de rétention. Ces étapes sont généralement effectuées à une température de 30 à 60°C.

Si des traitements chimiques ou autres sont nécessaires, par exemple au préalable, ils peuvent avoir lieu dans le pulpeur, ou dans d'autres installations séparées. Un exemple de tel traitement chimique est le blanchiment de la pâte à papier.

La pâte à papier stockée dans la cuve de rétention est alors mise en oeuvre sur une machine à papier et projetée sur une toile filtrante en mouvement. La toile filtrante est une toile filtrante reliée à une source de dépression permettant d'éliminer une grande partie d'eau par aspiration de celle-ci. La feuille est alors formée sur la toile filtrante avant d'être pressée entre des rouleaux et ensuite séchée au contact de rouleaux chauffés à la vapeur, à l'aide d'infrarouge, de microondes ou encore sur coussins d'air.

Selon la finition souhaitée, des étapes de finitions peuvent encore être effectuées telles que des teintures, ajout de d'argiles, de charges minérales, d'amidon ou encore d'autres substances chimiques avant le bobinage et coupage à dimension.

Le troisième type d'acteur concerne les fabricants de papier qui utilisent comme matière de départ, la matière cellulosique première (copeaux de bois et autres fibres) et produisent directement du papier ou du carton, en passant par la formation intermédiaire de pâte à papier.

Comme on peut le constater, les étapes du procédé de fabrication du papier sont très énergivores, en particulier l'étape de traitement mécanique et/ou chimique de la pâte à papier ainsi que le séchage ou encore les transports de pâte à papier en balles. De même, certaines étapes nécessitent l'utilisation de substances chimiques nocives pour l'environnement. Depuis de nombreuses années, des tentatives ont été effectuées pour réduire l'empreinte écologique de ces procédés en diminuant l'impact énergétique et la consommation de produits chimiques polluants.

Ainsi, de nombreuses substances chimiques ont été peu à peu remplacées par des enzymes. Par exemple, le désencrage est réalisé à l'aide de xylases et des amylases sont utilisées pour éliminer les résidus de colle dans la pâte à papier issue de papier et cartons recyclés.

En outre, de nombreuses publications ont fait leur apparition quant à l'utilisation des enzymes (cellulase et hemicellulase) dans le traitement mécanique pour déstructurer et déchirer les fibres de la pâte à papier.

Par exemple, le document US6267841 divulgue l'utilisation entre une étape de raffinage primaire et une étape de raffinage secondaire d'enzymes telles que des pectinases, xylanases, laccase, cellulase ou des combinaisons de celles-ci.

Ceci permet de réduire l'impact énergétique du deuxième raffinage grâce à l'action enzymatique qui contribue à la déstructuration des fibres dans la pâte à papier et réduit donc par conséquent l'apport d'énergie externe qui aurait été nécessaire sans l'utilisation d'enzymes.

Toutefois, il s'avère important de pouvoir contrôler le fonctionnement de ces enzymes qui peuvent perturber les étapes ultérieures puisque ces dernières ne sont pas éliminées entre les étapes du procédé.

Le document US5728263 divulgue que l'utilisation de dialdéhydes ou d'acétals permet d'inhiber la décomposition du peroxyde utilisé comme agent blanchissant par les enzymes utilisées (dans les étapes précédentes). Ceci permet donc d'obtenir une brillance appropriée du papier, mais également ceci permet de contrôler les effets des microorganismes (facteur considéré jusque là comme incontrolable).

Le document US20100126680 divulgue également que l'utilisation d'aldéhydes permet d'inhiber la décomposition du peroxyde utilisé comme agent blanchissant par les enzymes utilisées mais sous forme de polymère. Le polymère est introduit dans une quantité efficace pour contrôler les enzymes décomposant le peroxyde qui sont présentes dans la pulpe à blanchir.

Le document W02009079634 divulgue quant à lui l'utilisation de diverses enzymes telles que les amylases, cellulases, hémicellulases, protéases, lignocellulose en présence d'un co-facteur pour augmenter la conversion du substrat par l'enzyme. Le co-factuer utilisé peut être de diverse nature comme par exemple un acide nucléique, un oligo ou polynucléotide, un acide aminé, un peptide, une protéine, un anticoprs, un sucre, un hydrate de carbone, un monomère, un polymère, une petite molécule, une vitamine, un ion, un co-enzyme ou des combinaisons de ceux-ci. Ce document divulgue donc des polymères, en particulier un polyacrylate cationique permettant d'augmenter la catalyse enzymatique et donc la conversion du substrat, en l'occurrence, un polysaccharide pour former un biocarburant, par l'enzyme utilisée. Ce document ne divulgue donc aucun moyen d'inhiber l'activité enzymatique lorsque c'est nécessaire.

Dans les procédés papetiers, de nombreux paramètres sont contrôlés en permanence et les contraintes dictées par la nature du produit fini sont très strictes, laissant peu de marge de manoeuvre à la transformation des procédés existants pour les rendre moins énergivores. Ceci signifie que si aujourd'hui, il existe de nombreuses publications incitant à l'utilisation d'enzymes, par exemple dans le pulpeur, aucun procédé n'a pu être mis en oeuvre de manière efficace car l'activité enzymatique peut être trop importante au niveau du pulpeur ou persister dans les étapes ultérieures, ce qui nuit à la qualité du produit fini. En outre, si certains composés sont imaginables pour inhiber l'activité enzymatique, ils sont incompatibles avec les procédés papetiers car ils perturbent les caractéristiques du papier, du carton ou de la pâte à papier produite. C'est par exemple le cas de l'enseignement du document WO2006/104448 qui inhibe l'activité des enzymes xylanase par l'utilisation de Mn²⁺ ou partiellement en présence de Cu²⁺ et d'EDTA. Malheureusement, ces inhibitions ne sont pas réversibles.

Il existe donc toujours un besoin de pouvoir contrôler l'activité enzymatique lorsque c'est nécessaire, de manière relativement immédiate. Un exemple typique réside dans le fait que lorsque la pulpe est préparée dans le pulpeur, les enzymes peuvent être ajoutées à cet endroit. Comme elles ne sont pas inhibées actuellement, leur activité est maintenue en cas de temps de séjour prolongé dans ce pulpeur et elles persistent dans les étapes ultérieures de traitement et nuisent à la qualité du produit fini. En outre, si la machinerie doit être immédiatement arrêtée à cause d'un problème technique quelconque, il se peut que le temps de séjour dans le pulpeur ou dans la ou les cuves de rétention soit fortement augmenté, passant par exemple d'une trentaine de minutes à plusieurs heures. Ceci signifie que les enzymes présentes dégradent le substrat dans une mesure plus importante qu'attendu et que le contenu du pulpeur et de la cuve de rétention doivent être jeté. Le contenu de ces deux cuves ensembles est généralement de 50 à 200 m³ de pâte à papier ou de pulpe.

Il existe donc un besoin de pouvoir contrôler l'activité enzymatique en l'inhibant de manière très rapide tout en pouvant la réactiver rapidement. Il faut donc que l'inhibition ne soit pas irréversible, mais bien contrôlable.

L'invention a pour but de pallier les inconvénients de l'état de la technique en procurant un procédé permettant d'inhiber à la demande l'activité enzymatique et de la réactiver de manière rapide.

Pour résoudre ce problème, il est prévu suivant l'invention, un procédé tel qu'indiqué au début dans lequel ladite inhibition de l'activité enzymatique est une inhibition à la demande et réversible, en particulier lorsque la fabrication du papier, du carton ou de la pâte à papier doit être interrompue, effectuée par l'addition d'une quantité efficace de composé phosphonate.

En effet, il a été observé que la grande majorité des enzymes trouvant une utilité dans les procédés papetiers ont des cations comme co-facteur. Leur activité est donc médiée par des ions cationiques qui sont présents dans l'eau de pulpage (voire éventuellement complémentés dans l'eau de pulpage). Les composés phosphonates sont des composés séquestrants ou complexant qui complexent ces cations, les rendant donc indisponibles pour médier l'activité enzymatique. Ceci signifie que l'ajout d'une quantité efficace de composé phosphonate permet de rendre inaccessible les ions nécessaires au bon fonctionnement des enzymes et inhibe dès lors leur activité.

La quantité efficace sera donc fonction de nombreux paramètres comme la présence des ions dans l'eau de pulpage, la quantité de substrat à pulper, la quantité d'enzyme nécessaire, la nature de celui-ci et les différents additifs ou substances chimiques lorsqu'ils sont utilisés ainsi que de la nature du phosphonate utilisé.

Lorsque le procédé doit être stoppé, le phosphonate est ajouté en quantité efficace et complexe quasi immédiatement les cations présents dans le milieu empêchant ainsi l'activité enzymatique. De cette façon, lorsque le système est stoppé et que le phosphonate est ajouté, l'activité enzymatique est inhibée quasi immédiatement par manque de disponibilité de cations agissant comme co-facteur. Le phosphonate est un des rares composés inhibiteur d'enzymes dont la présence ne perturbe pas la qualité du papier produit ou du carton ou encore de la pâte à papier. Il peut de plus être utilisé efficacement en quantité sous stoéchiométrique, ce qui permet d'optimiser son dosage et de limiter les quantités injectées dans le procédé.

Avantageusement, le procédé selon l'invention comprend en outre, après ladite inhibition, une étape de réactivation enzymatique. De préférence, ladite réactivation enzymatique a lieu par un apport d'ions, en particulier par dilution par de l'eau, fournissant dès lors, à nouveau des cations agissant comme co-facteurs pour les enzymes, tandis que les phosphonates présents sont déjà à l'état complexé et amalgamés à la phase solide cellulosique. Si ledit phosphonate est un phosphonate sensible au chlore, ladite réactivation enzymatique peut également être réalisée par l'action de chlore qui réagit avec les phosphonates et les inactives, empêchant ainsi leur action inhibitrice sur les enzymes.

Dans une forme de réalisation préférentielle du procédé selon l'invention, ledit traitement de matières cellulosiques comprend une étape de pulpage de ladite matière cellulosique, avec obtention d'une pulpe et une étape de traitement mécanique et/ou chimique de ladite pulpe pour obtenir une pate à papier, et dans lequel ladite addition d'une ou de plusieurs enzymes a lieu lors de l'étape de pulpage. Cette partie du procédé concerne donc le deuxième type d'acteurs sur le marché ainsi que le troisième qui sont les deux types d'acteurs effectuant réellement du papier ou du carton. La matière cellulosique pulpée est donc soit la pâte à papier en ballot que les acteurs peuvent se procurer sur le marché, soit des papiers, journaux et cartons broyés ou déchiquetés issus du recyclage.

Cette étape de pulpage de ces matières cellulosique est en effet une des étapes de choix pour l'utilisation d'enzyme dans un procédé de fabrication de papier puisque cette étape permet de pré-traiter la pâte à papier avant l'étape de raffinage qui est particulièrement énergivore. Dans ce cas, les enzymes sont ajoutées de telles façons qu'elles soient actives lors du pulpage et favorisent le traitement mécanique et/ou chimique, par exemple lors de l'injection de la pâte à haute pression entre des roues cannelées d'un raffineur afin de déstructurer et déchirer les fibres ou encore lors d'une étape de blanchiment dans laquelle d'autres enzymes pourraient s'avérer utiles.

Dans une variante selon l'invention, ledit traitement de matières cellulosiques comprend une étape de pulpage de ladite matière cellulosique, avec obtention d'une pulpe et une étape de traitement mécanique et/ou chimique de ladite pulpe pour obtenir une pate à papier, et dans lequel ladite addition d'une ou de plusieurs enzymes a lieu lors de l'étape de traitement mécanique et/ou chimique. Comme précédemment, cette partie du procédé selon l'invention concerne donc le deuxième type d'acteurs sur le marché ainsi que le troisième qui sont les deux types d'acteurs effectuant réellement du papier ou du carton. En effet, dans certains cas, l'addition de la ou des enzymes peut être avantageusement effectuée lors de l'étape de traitement mécanique et/ou chimique, par exemple lors du désencrage ou lors du blanchiment. Notons au passage que dans certaines formes de réalisation, le traitement mécanique et/ou chimique a lieu pendant le pulpage.

Avantageusement, ladite étape de traitement mécanique et/ou chimique comprend une ou plusieurs étapes, en séquence ou conjointes, choisies parmi le raffinage mécanique, le broyage, le désencrage, le blanchiment, l'hydrolyse chimique, l'autoclavage, la séparation mécanique ou chimique, le déchiquetage, le lavage.

Comme on l'a mentionné ci-avant, généralement, le procédé selon l'invention comprend, après ledit traitement mécanique et/ou chimique, une étape de rétention.

Dans une forme de réalisation particulière, le procédé selon l'invention comprend, avant ledit traitement de matières cellulosiques, une étape de pulpage de matière cellulosique première, avec obtention d'une pulpe suivie d'une étape de séparation solide liquide pour obtenir une pâte à papier, et éventuellement d'une étape de mise en balles, dans lequel ladite addition d'une ou de plusieurs enzymes a lieu lors de l'étape de pulpage de ladite matière cellulosique première.

Cette partie du procédé concerne donc le traitement de la matière cellulosique première, c'est-à-dire par exemple des fibres et des copeaux de bois. Ce sont donc le premier et le troisième types d'acteurs qui sont concernés par cette partie du procédé selon l'invention. Il est en effet bien connu que le pulpage de la matière cellulosique première avec la raffinage sont des étapes également énergivores où les enzymes présentent une utilité reconnue. Toutefois, il existe également à ce stade un besoin de pouvoir contrôler l'activité enzymatique pour pouvoir limiter l'action des enzymes dans le temps si c'était nécessaire, par exemple dans le cas où un blocage ou un disfonctionnement mécanique se produit.

Dans une autre forme de réalisation du procédé selon l'invention qui concerne le premier et le troisième type d'acteurs sur le marché, le procédé comprend, avant ledit traitement de matières cellulosiques, une étape de pulpage de matière cellulosique première, avec obtention d'une pulpe suivie d'une étape de séparation solide liquide, pour obtenir une pate à papier, et éventuellement d'une étape de mise en balles, dans lequel ladite addition d'une ou de plusieurs enzymes a lieu lors de l'étape de séparation solide liquide. En effet, certaines enzymes peuvent améliorer les caractéristiques de la séparation solide liquide en ayant un effet sur l'agglomération des particules solides.

Avantageusement, ledit traitement de matière cellulosique comprend une étape de filtration d'une pate à papier à l'aide d'une toile filtrante pour fabriquer une feuille de papier ou carton, de séchage de la dite feuille de papier ou carton avant bobinage de celle-ci.

Avantageusement, ledit composé phosphonate est un phosphonate sensible au chlore choisi dans le groupe constitué du HEDP (acide 1-hydroxyéthylidène-1,1-diphosphonique), du DTPMP (acide diéthylènetriamine penta(méthylène) phosphonique), du DMMP (diméthyl méthylphosphonate) et du EDTMP (acide ethylènediamine tetra méthylène phosphonique). De manière plus préférentielle, le phosphonate est un HEDP.

De cette façon, l'utilisation de chlore qui est un composé autorisé dans les traitement papetiers, qui bien que susceptible d'avoir un impact environnemental, a l'énorme avantage de ne pas nuire aux qualités attendues du papier. Ces qualités attendues du papiers sont mesurées fréquemment pendant la production et doivent répondre à des normes très strictes de la part du fabricant. Quelques exemples de ces qualités attendues du papier formé sont la douceur, le grain, l'épaisseur, la résistance à l'étirement, la résistance à la déchirure, la transparence, teneur en eau.

De préférence, ladite une ou plusieurs enzymes est/sont choisies dans le groupe constitué des laccases, des catalases, des pectinelyases, des hydrolases dont des endocellulases, des exocellulases, des hemicellulases, des xylanases, des endoglucanases, des beta-glucanases, des arabanases, des mananases, des lipases, des amylases, des proteases, des polygalacturonases, des pectinesterases.

D'autres formes de réalisation du procédé suivant l'invention sont indiquées dans les revendications annexées.

L'invention a aussi pour objet une trousse comprenant une ou plusieurs enzymes et un phosphonate, de préférence, un phosphonate sensible au chlore choisi dans le groupe constitué du HEDP (acide 1-Hydroxy éthylidène-1,1-disphosphonique, du DTPMP (acide diéthylènetriamine penta,méthylène phosphonique), du DMMP (diméthyl méthylphosphonate) et de l'EDTMP (acide ethylènediamine tetra méthylène phosphonique)

D'autres formes de réalisation de la trousse sont mentionnées dans les revendications annexées.

L'invention a aussi pour objet une utilisation d'un phosphonate pour inhiber l'activité enzymatique d'une ou de plusieurs enzymes dans un procédé de fabrication de papier, de carton ou de pâte à papier. Dans cette utilisation selon l'invention, le phosphonate est de préférence un phosphonate sensible au chlore choisi dans le groupe constitué du HEDP (acide 1-hydroxy éthylidène-1,1-diphosphonique, du DTPMP (acide diethylènetriamine penta,méthylène phosphonique), du DMMP (diméthyl méthylphosphonate) et de l'EDTMP (acide éthylènediamine tetra(méthylène phosphonique).

D'autres formes de réalisation de l'utilisation suivant l'invention sont indiquées dans les revendications annexées.

D'autres caractéristiques, détails et avantages de l'invention ressortiront de la description donnée ci-après, à titre non limitatif et en faisant référence aux exemples.

### Exemple 1

### Essai pilote

Une pâte à papier composée de : 50% de fibres courtes de feuillus et de 50% de fibres longues de résineux a été pulpée en présence d'eau dans une cuve. Piloté en présence de 0,06% (en poids par rapport au poids de la pâte sèche) d'enzymes composés d'exo-cellulases et endo-cellulase.

Le mélange a été agité pendant 45 minutes avant d'être raffiné dans une pile VALLEY pour obtenir un degré d'égouttage de 48°SR (degré SHOPPER RIEGLER).

Ensuite, on a ajouté 0,1 % d'inhibiteur constitué d'un phosphonate HEDP ; Le pourcentage est un pourcentage en poids par rapport au poids de pâte sèche.

La préparation fibreuse a ensuite été encore agitée pendant 1 heure et le degré de raffinage de la pâte a été mesuré à 48°SR.

L'introduction de l'inhibiteur a permis de conserver des caractéristiques identiques de la pâte malgré la présence d'enzymes.

### Exemple 2

### Essai industriel

Une pâte à papier composée de 75 % de fibres courtes de feuillus et de 25 % de fibres longues de feuillus ont été pulpée en présence d'eau dans une cuve de rétention, en présence de 0,021 % d'enzymes (en poids par rapport au poids de pâte sèche)

La puissance de raffinage nécessaire pour raffiner cette pâte à papier a été réduite de 35% par rapport à la puissance nécessaire pour une pâte à papier identique mais sans enzyme.

Les caractéristiques mécaniques du papier ont été obtenues selon le cahier des charges du papetier.

Lors d'un arrêt imprévu, 0,05% d'inhibiteur constitué de HEDP a été introduit, le pourcentage est en poids par rapport au poids de pâte sèche dans les cuviers de pâte concentrée (à 3,5% de concentration).

Le procédé a été arrêté pendant 6 heures.

La production de papier a été redémarrée avec les caractéristiques mécaniques du papier identiques à celles d'avant l'arrêt.

Les caractéristiques de la pâte stockée dans les cuviers pendant l'arrêt n'ont pas eu d'incidence sur la qualité au papier qui était toujours conforme au cahier des charges imposé par le papetier.

Ensuite, les enzymes ont été réactivées lors de la production et on a observé qu'il n'ya pas eu d'augmentation de puissance de raffinage de la pâte neuve pendant la production avec enzymes.

Comme on peut donc le constater, le procédé selon l'invention permet d'arrêter et de reprendre à la demande l'activité des enzymes.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Procédé de traitement de matières cellulosiques pour fabriquer du papier, du carton ou de la pâte à papier comprenant les étapes suivantes :
- addition d'une ou de plusieurs enzymes ayant une activité dans le traitement susdit desdites matières cellulosiques,
- inhibition de ladite activité enzymatique,
**caractérisé en ce que** ladite inhibition de l'activité enzymatique est une inhibition à la demande et réversible, en particulier lorsque la fabrication du papier, du carton ou de la pâte à papier doit être interrompue, effectuée par l'addition d'une quantité efficace d'un composé phosphonate.

2. Procédé selon la revendication 1, comprenant en outre, après ladite inhibition, une étape de réactivation enzymatique.

3. Procédé selon la revendication 2, dans lequel ladite réactivation enzymatique a lieu par un apport d'ions, en particulier par dilution par de l'eau.

4. Procédé selon l'une quelconque des revendications 1 à 3, dans lequel ledit traitement de matières cellulosiques comprend une étape de pulpage de ladite matière cellulosique, avec obtention d'une pulpe et une étape de traitement mécanique et/ou chimique de ladite pulpe pour obtenir une pate à papier, et dans lequel ladite addition d'une ou de plusieurs enzymes a lieu lors de l'étape de pulpage.

5. Procédé selon l'une quelconque des revendications 1 à 3, dans lequel ledit traitement de matières cellulosiques comprend une étape de pulpage de ladite matière cellulosique, avec obtention d'une pulpe et une étape de traitement mécanique et/ou chimique de ladite pulpe pour obtenir une pate à papier, et dans lequel ladite addition d'une ou de plusieurs enzymes a lieu lors de l'étape de traitement mécanique et/ou chimique.

6. Procédé selon la revendication 4 ou 5, dans lequel ladite étape de traitement mécanique et/ou chimique comprend une ou plusieurs étapes, en séquence ou conjointes, choisies parmi le raffinage mécanique, le broyage, le désencrage, le blanchiment, l'hydrolyse chimique, l'autoclavage, la séparation mécanique ou chimique, le déchiquetage, le lavage.

7. Procédé selon l'une quelconque des revendications 4 à 6, comprenant, après ledit traitement mécanique et/ou chimique, une étape de rétention.

8. Procédé selon l'une quelconque des revendications précédentes comprenant, avant ledit traitement de matières cellulosiques, une étape de pulpage de matière cellulosique première, avec obtention d'une pulpe suivie d'une étape de séparation solide liquide pour obtenir une pâte à papier, et éventuellement d'une étape de mise en balles, dans lequel ladite addition d'une ou de plusieurs enzymes a lieu lors de l'étape de pulpage de ladite matière cellulosique première.

9. Procédé selon l'une quelconque des revendications 1 à 7 comprenant, avant ledit traitement de matières cellulosiques, une étape de pulpage de matière cellulosique première, avec obtention d'une pulpe suivie d'une une étape de séparation solide liquide, pour obtenir une pate à papier, et éventuellement d'une étape de mise en balles, dans lequel ladite addition d'une ou de plusieurs enzymes a lieu lors de l'étape de séparation solide liquide.

10. Procédé selon l'une quelconque des revendications 1 à 9, dans lequel ledit traitement de matière cellulosique comprend une étape de filtration d'une pate à papier à l'aide d'une toile filtrante pour fabriquer une feuille de papier ou carton, de séchage de la dite feuille de papier ou carton avant bobinage de celle-ci.

11. Procédé selon l'une quelconque des revendications 1 à 10, dans lequel ledit composé phosphonate est un phosphonate sensible au chlore choisi dans le groupe constitué de l'acide 1-hydroxy éthylidène-1,.1-diphosphonique (HEDP), de l'acide diéthylènetriamine penta. méthylène phosphonique (DTPMP), du diméthyl methylphosphonate (DMMP) et de l'acide ethylènediamine tetra(méthylène phosphonique (EDTMP).

12. Procédé selon la revendication 11, dans lequel ledit phosphate sensible au chlore, est activé par une présence de chlore.

13. Procédé selon l'une quelconque des revendications précédentes, dans lequel ladite une ou plusieurs enzymes est/sont choisies dans le groupe constitué des laccases, des catalases, des pectinelyases, des hydrolases dont des endocellulases, des exocellulases, des hemicellulases, des xylanases, des endoglucanases, des beta-glucanases, des arabanases, des mananases, des lipases, des amylases, des proteases, des polygalacturonases, des pectinesterases.

14. Trousse pour mettre en oeuvre le procédé selon l'une quelconque des revendications précédentes, comprenant une ou plusieurs enzymes et un phosphonate, de préférence, un phosphonate sensible au chlore choisi dans le groupe constitué de l'acide 1-hydroxy éthylidène-1,1-diphosphonique (HEDP), de l'acide diéthylènetriamine penta méthylène phosphonique (DTPMP), du diméthyl methylphosphonate (DMMP) et de l'acide ethylènediamine tetra(méthylène phosphonique (EDTMP).

15. Utilisation d'un phosphonate pour inhiber l'activité enzymatique d'une ou de plusieurs enzymes dans un procédé de fabrication de papier, de carton ou de pâte à papier.

16. Utilisation selon la revendication 14, dans lequel le phosphonate est un phosphonate sensible au chlore choisi dans le groupe constitué de l'acide 1-hydroxy éthylidène-1,1-diphosphonique (HEDP), de l'acide diéthylènetriamine penta(méthylène phosphonique (DTPMP), du diméthyl methylphosphonate (DMMP) et de l'acide ethylènediamine tetra(méthylène phosphonique (EDTMP).
